# EUROPEAN PATENT APPLICATION

(11) **EP 2 322 602 A2**
(43) Date of publication of application: **18.05.2011**
(21) Application number: 09803111.5
(22) Date of filing: 17.07.2009
(51) Int. Cl.: C12N 5/0775

(54) **METHOD FOR SEPARATING HIGHLY ACTIVE STEM CELLS FROM HUMAN STEM CELLS AND HIGHLY ACTIVE STEM CELLS SEPARATED THEREBY**

(30) Priority: 28.07.2008 KR 20080073383
(71) Applicant: Seoul National University Hospital, Seoul 110-744 (KR)
(72) Inventor: KIM, Hyo Soo, Seoul 137-930 (KR); LEE, Eun Ju, Seoul 136-779 (KR); KANG, Hyun Jae, Seoul 110-054 (KR)
(74) Representative: Goddar, Heinz J.
(86) International application number: PCT/KR2009/003954
(87) International publication number: WO 2010/013906

(57) **Abstract**

The present invention relates to a method for separating highly active stem cells from human stem cells, the highly active stem cells separated by the method, a cell therapeutic agent containing the stem cells, and a medium for separating the highly active stem cells from stem cells containing a specific cytokine. According to the present invention, the method is useful for separating the highly efficient stem cells from mesenchymal stem cells of various origins. Further, the method is very useful in developing a cell therapeutic agent of high efficiency because the method can be applicable to stem cells of various origins which are cultured under different conditions. Senescent stem cells increased by several passage times in vitro can be effectively sorted out, so the method can be used for reactivating the stem cells.

## Description

### FIELD OF THE INVENTION

The present invention relates to a method for separating highly active stem cells from human stem cells, the stem cells separated by the method, a cell therapeutic agent containing the stem cells, and a specific medium.

### BACKGROUND OF THE INVENTION

Stem cells are capable of differentiating into a variety of cells constituting tissues of an organism, and generally refer to undifferentiated cells obtainable from an embryo, a fetus and each tissue of an adult body. Stem cells differentiate into specialized cells by a specific differentiation stimulus (environment); are capable of proliferation (expansion) by producing identical cells through cell division (self-renewal), unlike the differentiated cells whose cell division have been ceased; and are characterized by their plasticity of differentiation that they can differentiate into other cells under a different environment or by a differentiation stimulus.

Stem cells are largely divided into embryonic stem cells (ES cells) which are obtainable from an embryo and are pluripotent, i.e., are capable of differentiating into all cell types; and multipotent adult stem cells obtainable from each tissue. The inner cell mass of an early embryo at the blastocyst stage forms a fetus in the future and embryonic stem cells isolated from the inner cell mass theoretically have a totipotency of being capable of differentiating into cells of every type of tissues constituting an organism. Namely, embryonic stem cells are undifferentiated cells capable of indefinite proliferation, can differentiate into all cell types, and can be inherited to the next generation through the preparation of germ cells, unlike the adult stem cells.

Human embryonic stem cells are prepared by isolating and culturing the inner cell mass of a human blastocyst and, currently, all the human embryonic stem cells prepared worldwide have been derived from the frozen embryos remained after the sterility treatment. Such cells are characterized in that they can differentiate into the cells of every type of tissues due to their pluripotency of differentiating into all cell types, can be cultured in an immortal and undifferentiated state, and can be inherited to the next generation through the preparation of germ cells (Thomson et al., Science, 282: 1145-1147, 1998; and Reubinoff et al., Nat. Biotechnol., 18: 399-404, 2000).

Various approaches for using pluripotent human embryonic stem cells as a cell therapeutic agent have not yet completely overcome the problems such as carcinogenesis and immunological rejection.

Recently, mesenchymal stem cells reported to have an immunomodulatory activity have been presented as an alternative for solving such problems. Mesenchymal stem cells are multipotent cells capable of differentiating into adipocytes, osteocytes, chondrocytes, myocytes, neurocytes, cardiomyocytes, etc. and have been reported to have an activity for regulating immune responses. They can be separated from a variety of tissues, however, their capacity and cell surface markers are different from each other according to their origins. Therefore, the mesenchymal stem cells are currently defined in terms of differentiation capability into osteocytes, chondrocytes and myocytes, spiral form of the cells, and basic cell surface markers of SH2(+), SH3(+), CD34(-) and CD45(-).

Minimal number of cells necessary in the cell therapy or regenerative medicine is about 1 x 10⁹. However, a further amount of cells are necessary to carry out the experiments for setting conditions and establishing a standard. In the case of existing mesenchymal stem cells derived from various sources, at least 10 passages are required to obtain such amount of cells. Then, the cells would become aged and modified, which would make them inadequate for use in the therapy. This is one of the problems that involved in the current culture system for mesenchymal stem cells. Further, even when the conditions and the standard have been set by employing the cells, the cells would become depleted before they are used in the therapy. Under the circumstances, there may be no choice but to use mesenchymal stem cells from others, which necessitates additional experiments according to the use of different cells. Therefore, a novel method is required to make it possible to use the existing mesenchymal stem cells as a therapeutic agent with solving the above problem.

Korean Patent Publication No. 2008-3418 teaches a method for inducing embryonic stem cells to pancreas cells, and Korean Patent No. 10-593397 discloses a wound healing or wound healing promotive agent, or cell therapeutic agent containing mesenchymal stem cells and/or substance P.

### SUMMARY OF THE INVENTION

Therefore, it is an object of the present invention, which is designed to meet the needs presented above, to provide a method of producing cells required for cell therapy and regenerative medicine, the method overcoming the limitation of previous *ex vivo* expansion of human stem cells, maintaining rehabilitation of senescent cells, and being utilized in cells having various genetic backgrounds.

In accordance with one aspect of the present invention, there is provided a method for separating highly active stem cells from human stem cells.

Further, in accordance with other aspect of the present invention, there is provided highly active stem cells separated by said method.

Furthermore, in accordance with other aspect of the present invention, there is provided a cell therapeutic agent comprising said stem cells.

Still further, in accordance with other aspect of the present invention, there is provided a particular medium for separating highly active stem cells from human stem cells.

According to the present invention, the method is useful in developing a cell therapeutic agent because the method is applicable to previously established mesenchymal stem cells, as well as currently available other human mesenchymal stem cells of various origins which are cultured under different conditions, as sources of mesenchymal stem cells.

### BRIEF DESCRIPTION OF THE DRAWINGS

The above and other objects and features of the present invention will become apparent from the following description of the invention, when taken in conjunction with the accompanying drawings, which respectively show:
Fig. 1: the photograph showing spheres formed by the inventive method.
Fig. 2: the cell cycle analysis by fluorescence activated cell sorting (FACS) for stem cells separated by the inventive method, which confirms the increase of S (synthetic) phase indicating vigorous proliferation.
Fig. 3: the cytokine secretion assay of stem cells separated by the inventive method, which shows the significant increases of IL-6, GM-CSF, VEGF, HGF, IL-8, IFN-g, and bGFG.
Fig. 4: the enhancement of stemness by the inventive method, which shows the increases of OCT-4 and E-cadherin as pluripotent cell markers.
Fig. 5: the diagram showing the process of the inventive method.

### DETAILED DESCRIPTION OF THE INVENTION

Hereinafter, the present invention is described in detail.

To accomplish the object of the present invention, the present invention provides a method for separating highly active stem cells from human stem cells, comprising culturing human stem cells to form spheres. More particularly, the method comprises the steps of:
(a) culturing human stem cells to form spheres; and
(b) separating the spheres from other non-sphere forming cells.

In the method of the present invention, the step (a) may be carried out in a bovine serum-free medium by employing a low-attachment Petri dish. The bovine serum-free medium may preferably comprise bovine serum-free DMEM (Dulbecco's Modified Eagle's Medium) and 20% SR (Serum Replacement). The low-attachment Petri dish refers to a Petri dish leading to attachment of adhesive growth cells in an efficiency of less than 5%.

Preferably, the step (a) may be carried out by treating human stem cells with a protease and suspending the treated cells in a medium comprising bovine serum-free DMEM (Dulbecco's Modified Eagle's Medium) and 20% SR for 20~28 hours, preferably for 24 hours by employing a low-attachment Petri dish.

An example of the protease is trypsin, but not limited thereto.

The step (b) may be carried out by using a strainer, but not limited thereto, for separating the spheres from other non-sphere forming cells.

The stem cells are preferably mesenchymal stem cells.

The term "stem cells" as used herein refers to master cells which can regenerate unlimitedly to form specialized cells of tissues and organs. A stem cell is a multipotent or pluripotent cell. A stem cell divides into two daughter stem cells, or into one daughter stem cell and one transit cell, and subsequently is proliferated into a mature and complete type of cell of tissue.

The term "mesenchymal stem cell" as used herein can be differentiated into osteoblasts, chondrocytes, myocytes, and others, and characterized by its shape of whirlpool and its expression levels of standard cell surface markers, SH2(+), SH3(+), CD34(-), and CD45(-).

The method of the present invention consists of culturing human stem cells in a bovine serum-free medium by employing a low attachment rate of Petri dish to form spheres. In said step, the bovine serum-free medium used for forming spheres is a combination of DMEM standard medium and Serum Replacement (SR). It will be readily appreciated by the skilled in the art that a variety of cytokines may be added thereto based on the situation.

The Serum Replacement (SR) used in the method of the present invention has been employed instead of FBS, in human embryonic stem cell culture for the purpose of removing factors derived from animal. In the present invention, the SR was used for nutrition supply and suspension, instead of FBS which supports the attachment rate of adhesive cells.

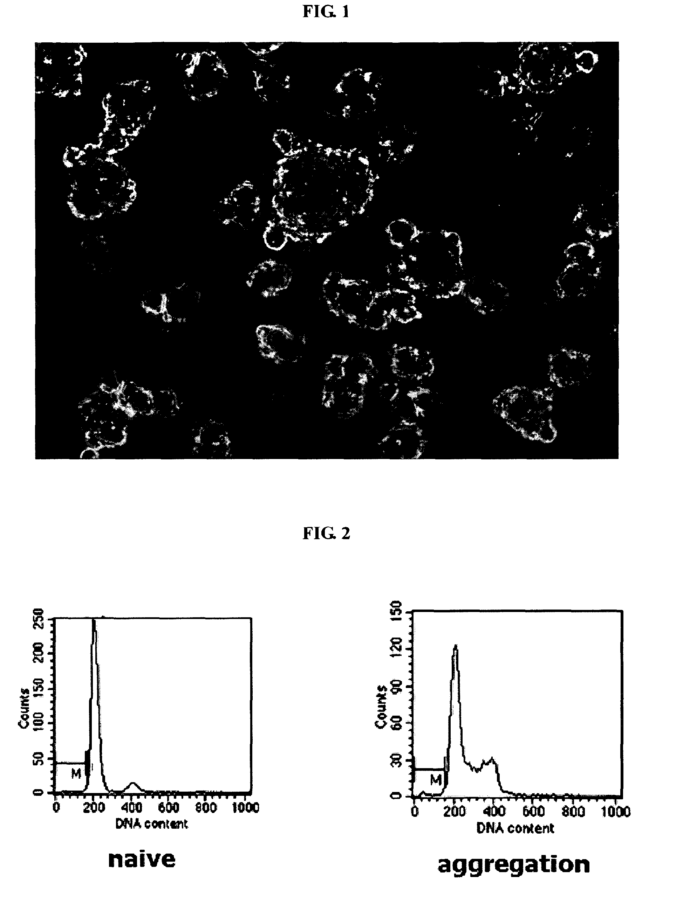

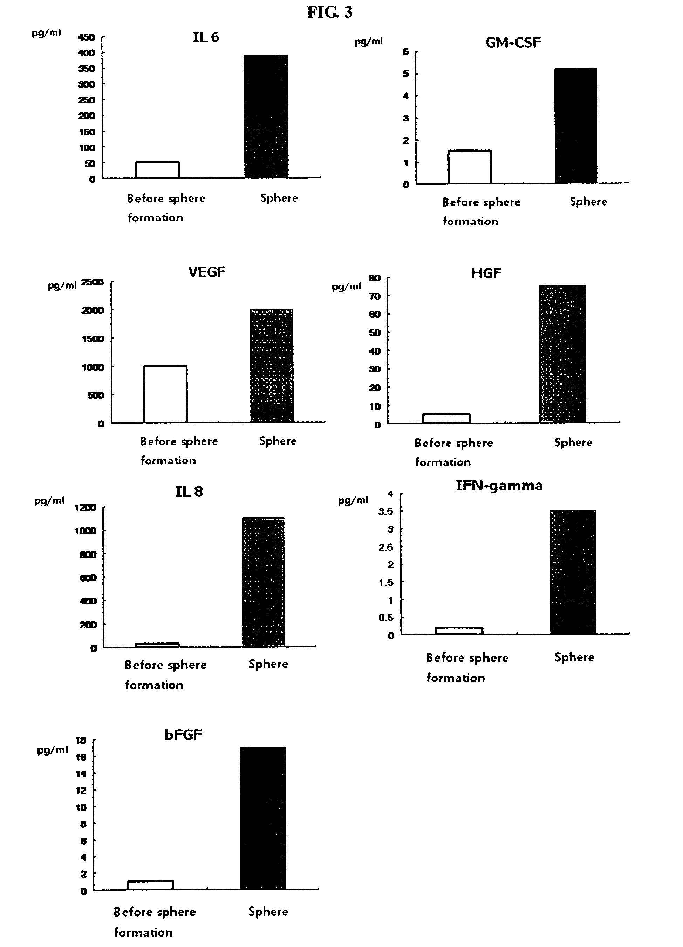

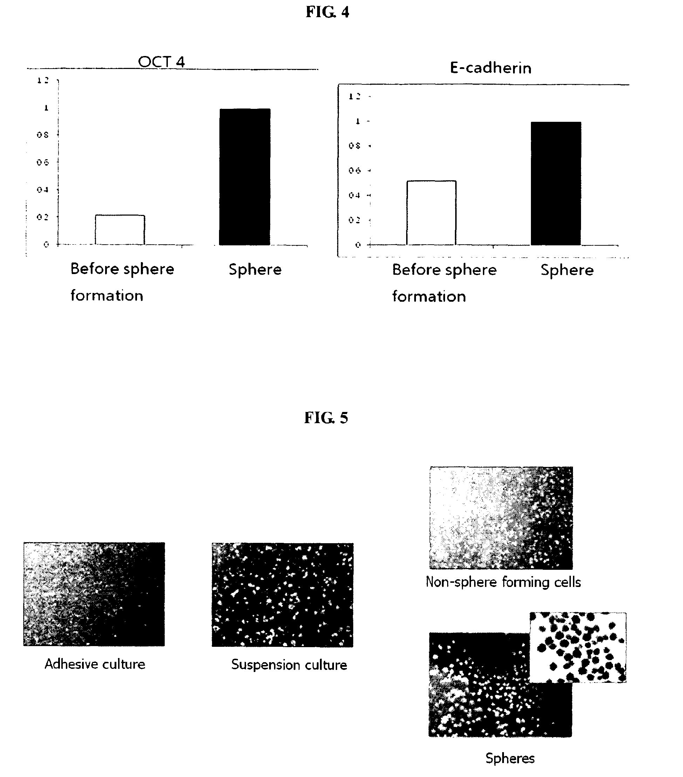

The present invention also provides highly active stem cells separated by said method. The separated stem cells exhibit the enhancement of cytokine secretion, and the increases of expression of stem cell genes, cell survival and cell growth (*see,* Figs. 2 to 4).

The stem cells separated by the inventive method showed similar results with that of stem cells of different origins, which was confirmed by Fluorescence Activated Cell Sorting (FACS), Enzyme-Linked Immunosorbent Assay (ELISA), and Real-Time Polymerization Chain Reaction (PCR).

Further, the present invention provides a cell therapeutic agent comprising the stem cells separated by the inventive method.

In particular, the cell therapeutic agent may be used for generating adipocytes, osteocytes, chondrocytes, myocytes, neurocytes, cardiomyocytes, and others.

The term "cell therapeutic agent" as used herein refers to a drug for treatment, diagnosis, and prevention (U.S. FDA guidance) comprising cells or tissues prepared from humans via isolation, culture and specialized manipulations, more particularly, to a drug for treatment, diagnosis, and prevention prepared by any process including proliferating or selecting autologous, homologous or heterologous cells *ex vivo,* or modifying the biological characteristics of cells, so as to restore the function of cells or tissues. Cell therapeutic agents are largely classified into somatic cell and stem cell therapeutic agents based on the differentiation levels, and the present invention is directed to a stem cell therapeutic agent.

The present invention also provides a medium for separating highly active stem cells from stem cells containing SR, etc. The medium may preferably contain a bovine serum-Free DMEM (Dulbecco's Modified Eagle's Medium) and 20% SR. The stem cells are preferably mesenchymal stem cells.

It can be appreciated by the skilled in the art that the medium can further comprise other supportive ingredients to separate the highly efficient stem cells from the stem cells.

The present invention is further defined in the following Examples. It should be understood that these Examples, while indicating preferred embodiments of the invention, are given by way of illustration only and are not intended to limit the scope of the invention.

### Example

### Sphere formation

Human stem cells maintained *ex vivo* were treated with a proteinase (0.25% trypsin/EDTA) and suspended in a medium comprising bovine serum-free DMEM (Dulbecco's Modified Eagle's Medium) and 20% SR (GIBCO) for 24 hours by employing a low attachment Petri dish (SPL).

### Sphere separation

The spheres formed by suspending the cells for 24 hours were separated from other non-sphere forming cells using a strainer.

### Characteristic analysis of the separated cells

1) Quantative gene expression analysis using Real-Time Polymerization Chain Reaction
   cDNA was synthesized from total RNA of the separated cells, and subjected to Real time PCR with specific primers related to stem cell gene.
2) Cytokine secretion assay by antigen-antibody reaction
   The secretion capability of various cytokines was analyzed using an antigen in the cell culture from the separated cells.
3) Cell cycle analysis by fluorescence activated cell sorting (FACS)
   The cell cycle of the separated cells was confirmed by a nuclear staining.

### Example 1: Separation of highly efficient stem cells from mesenchymal stem cells of human umbilical cord blood origin

Highly efficient mesenchymal stem cells were separated from mesenchymal stem cells originated in human umbilical cord blood according to the inventive method. The stem cells maintained *ex vivo* were treated with a proteinase (0.25% trypsin/EDTA) and suspended in a medium comprising bovine serum-free DMEM (Dulbecco's Modified Eagle's Medium) and 20% SR for 24 hours by employing a low attachment Petri dish. The spheres formed by suspending the cells for 24 hours were separated from other non-sphere forming cells using a strainer. cDNA was synthesized from total RNA of the separated cells, and subjected to Real-Time polymerization chain reaction with specific primers related to stem cell gene to confirm the gene expression levels related to the stem cell marker. The secretion capability of various cytokines was analyzed using an antigen in the cell culture medium from the separated cells, and cell cycle of the separated cells was confirmed by a nuclear staining.

It has found that the gene expression levels related to the stem cells after the sphere formation were increased and S (synthetic) phase was significantly increased. Also the angiogenesis and growth-related cytokines were significantly increased.

### Example 2: Separation of highly efficient stem cells from mesenchymal stem cells of human embryonic stem cell origin

Highly efficient mesenchymal stem cells were separated from mesenchymal stem cells originated in human embryonic stem cells according to the inventive method. The stem cells maintained *ex vivo* were treated with a proteinase (0.25% trypsin/EDTA) and suspended in a medium comprising bovine serum-free DMEM (Dulbecco's Modified Eagle's Medium) and 20% SR for 24 hours by employing a low attachment Petri dish. The spheres formed by suspending the cells for 24 hours were separated from other non-sphere forming cells using a strainer. cDNA was synthesized from total RNA of the separated cells, and subjected to Real-Time Polymerization Chain Reaction with specific primers related to stem cell gene to confirm gene expression related to the stem cell marker. The secretion capability of various cytokines was analyzed using an antigen in the cell culture medium from the separated cells, and cell cycle of the separated cells was confirmed by a nuclear staining.

It has found that the gene expression levels related to the stem cells after the sphere formation were increased and S (synthetic) phase was significantly increased. Also the angiogenesis and growth-related cytokines were significantly increased.

While the invention has been described with respect to the above specific embodiments, it should be recognized that various modifications and changes may be made to the invention by those skilled in the art which also fall within the scope of the invention as defined by the appended claims.

## Claims

1. A method for separating highly active stem cells from human stem cells, comprising the steps of:
(a) culturing human stem cells to form spheres; and
(b) separating the spheres from other non-sphere forming cells.

2. The method of claim 1, wherein step a) is carried out in a bovine serum-free medium by employing a low attachment Petri dish.

3. The method of claim 2, wherein the bovine serum-free medium comprises bovine serum-free DMEM (Dulbecco's Modified Eagle's Medium) and 20% SR (serum replacement).

4. The method of claim 1, wherein step a) is carried out by treating human stem cells with a proteinase and suspending the treated cells in a medium comprising bovine serum-free DMEM (Dulbecco's Modified Eagle's Medium) and 20% SR for 20 to 28 hours by employing a low attachment Petri dish.

5. The method of claim 1, wherein the stem cells are mesenchymal stem cells.

6. A highly active stem cell separated by the method of any one of claims 1 to 5.

7. A cell therapeutic agent for generating adipocytes, osteocytes, chondrocytes, myocytes, neurocytes or cardiomyocytes, comprising the highly active stem cell of claim 6.

8. A medium for separating highly active stem cells from human stem cells, comprising bovine serum-free DMEM (Dulbecco's Modified Eagle's Medium) and 20% SR.
